# FASCICULE DE BREVET EUROPEEN

(11) **EP 2 838 427 B1**
(45) Date de publication et mention de la délivrance du brevet: **22.02.2017**
(21) Numéro de dépôt: 13720458.2
(22) Date de dépôt: 08.04.2013
(51) Int. Cl.: A61B 5/11, G08B 21/04, A61B 5/00

(54) **PIÈCE DE REVÊTEMENT DE SOL POUR LA DÉTECTION DE CHUTES**
BODENBELAGELEMENT ZUR ERKENNUNG VON STÜRZEN
FLOOR COVERING ITEM FOR DETECTING FALLS

(30) Priorité: 19.04.2012 FR 1201156; 08.03.2013 FR 1352109
(43) Date de publication de la demande: 25.02.2015
(73) Titulaire: ABCD INNOVATION, 75017 PARIS (FR)
(72) Inventeur: DESGORCES, Claude, F-33470 Gujan Mestras (FR)
(74) Mandataire: Palacci, Jeremie
(86) Numéro de dépôt international: PCT/FR2013/050760
(87) Numéro de publication internationale: WO 2013/156707

(56) Documents cités:
- EP-A1- 2 050 426
- WO-A1-01/08755

## Description

L'invention concerne le domaine des pièces de revêtements de sol, et plus particulièrement des revêtements de sol capables de détecter la chute d'une personne.

Tous les pays développés connaissent un vieillissement de leur population qui s'accélère. Ce vieillissement se traduit par un très fort accroissement du nombre de personnes âgées de 60 ans et plus. Cette situation crée un véritable défi dans le domaine de la santé publique. Elle engendre aussi de sérieux problèmes de gestion de la dépendance des personnes âgées.

En effet, les personnes âgées voient leur espérance de vie augmenter tous les ans. Par ailleurs, l'évolution des structures sociales fait que ces personnes vivent de plus en plus seules, ou dans des structures spécialisées.

Pour les personnes vivant seules, cet isolement est un problème aigu car l'incapacité à prévenir les secours en cas de chute peut conduire au décès. Dans le cas de structures spécialisées, la détection des chutes est également très importante si l'on veut conserver des équipes soignantes de taille raisonnable, et un coût réaliste de la fourniture de soins, sans pour autant risquer des litiges en responsabilité pour défaut de surveillance.

La prise de conscience croissante de ces problèmes a suscité la réalisation d'études qui ont montrées que plus de 7 500 personnes meurent chaque année en France des suites d'une chute non détectée à temps, ou des conséquences d'une chute gérée tardivement.

La Demanderesse a proposé un dispositif permettant de détecter de telles chutes et prenant par exemple la forme de tapis ou de sol complet aux dimensions standards. Ce dispositif est décrit dans la demande de brevet français FR 11/02512. Un tel dispositif peut être utilisé dans un hôpital ou dans une maison de retraite par exemple, et repose sur la transformation d'une pression causée par une chute en un signal électrique associé à un emplacement connu, afin de détecter cette chute. Ces dispositifs peuvent recouvrir l'intégralité des sols d'un établissement de santé, ou du moins leurs parties destinées à être fréquentées par des patients âgés.

EP 2 050 426 décrit une pièce de carrelage munie d'une pluralité de capteurs de pression capacitifs, deux connecteurs agencés dans des côtés opposés de chaque pièce, un microcontrôleur pour identifier quelle pièce transmet des information de présence.

De tels dispositifs sont appréciés et satisfont à leurs objectifs. Néanmoins, leur création a fait apparaître un nouveau besoin. La Demanderesse a constaté que la conception et la fabrication de tels dispositifs sont réalisées sans tenir compte des spécificités des lieux dans lesquels ils sont voués à être installés. Cela limite l'adéquation des « tapis sensitifs » avec leur environnement de destination, c'est-à-dire des chambres, couloirs et salles communes. En effet, la diversité des formes et surfaces des sols à équiper est importante. En conséquence, soit il est proposé un panel de formes et surfaces de tapis et de revêtements très variées, voire sur mesure, à des coûts élevés, soit il est proposé un panel restreint de modèles moins adaptables mais à coût limité. Cela n'est pas entièrement satisfaisant.

D'autre part, ces revêtements de sol sensitifs impliquent l'utilisation de câblage électrique en quantité sensiblement proportionnelle à la surface du revêtement. Lorsque des surfaces importantes doivent être équipées, telles que des couloirs de maisons de santé, le câblage électrique devient un paramètre critique de par son encombrement et sa quantité. L'augmentation des coûts associée au câblage électrique rend d'autant plus rentables les efforts visant à limiter son utilisation, tant du point de vue de la quantité de câbles nécessaire, que du point de vue du temps nécessaire pour l'assembler.

L'invention vient améliorer la situation.

À cet effet, l'invention propose une pièce de revêtement pour la détection de chute comprenant
- un corps délimité par des bords,
- une pluralité de capteurs de pression répartis selon une géométrie choisie dans le corps,
- une unité de traitement reliée à certains au moins des capteurs de pression et agencée pour collecter des informations d'état de ces capteurs de pression, et
- au moins une première prise et une seconde prise, chacune reliée à l'unité de traitement, disposée au voisinage d'un bord et agencée pour pouvoir être reliée à une prise d'une autre pièce similaire.

L'unité de traitement est agencée pour
- associer des informations de localisation tirées desdites informations d'état à des informations de localisation de la pièce,
- recevoir, par l'intermédiaire de la première prise, des informations en provenance d'une première autre pièce similaire, et
- émettre, par l'intermédiaire de la seconde prise, les informations associées et/ou lesdites informations reçues vers une seconde autre pièce similaire.

En outre, une telle pièce peut présenter les caractéristiques optionnelles suivantes :
- la couche de surface et la couche inférieure comprennent chacun un film portant, sur des faces en regard, chacun un jeu de lames conductrices, les lames conductrices étant sensiblement parallèles au sein de chaque jeu, et les jeux de lames conductrices étant agencés de sorte que les lames conductrices de jeux distincts sont sensiblement perpendiculaires entre elles et se croisent au niveau des évidements de la couche intermédiaire, de manière à former à chaque fois un capteur de pression,
- le corps est de forme générale rectangulaire ou carrée, l'unité de traitement étant disposée dans un coin du corps.

Selon un autre aspect, l'invention concerne un kit comprenant un lot de plusieurs pièces. Le kit peut comprendre en outre une unité centrale pour la détection de chute incluant
- une prise propre à être reliée à au moins une prise d'au moins une pièce,
- un processeur relié à ladite prise et agencé pour
   - collecter des informations d'état de capteurs de pression associées à des informations de localisation desdits capteurs de pression couplées à des informations d'identification de pièces,
   - calculer à partir des informations collectées une matrice d'état d'un ensemble de pièces interconnectées,
   - détecter un état d'alerte à partir de la matrice d'état,
   - émettre un signal d'alerte sélectionné en fonction de l'état d'alerte détecté.

D'autres caractéristiques et avantages de l'invention apparaîtront mieux à la lecture de la description qui suit, tirée d'exemples donnés à titre illustratif et non limitatif, tirés des dessins sur lesquels :
- la figure 1 représente une vue schématique du dessus d'une pièce de revêtement selon l'invention, dans laquelle une couche de surface de la pièce a été retirée,
- la figure 2 représente un schéma électrique simplifié d'une portion de détection de la pièce de la figure 1,
- la figure 3 représente un schéma électrique simplifié d'un élément de la figure 2 et son environnement,
- la figure 4 représente un schéma électrique simplifié d'une portion de détection de la pièce de la figure 1 selon un autre mode de réalisation,
- la figure 5 représente un schéma électrique simplifié d'un élément de la figure 4 et son environnement,
- la figure 6 représente un éclaté d'une portion de la pièce de la figure 1,
- la figure 7 représente une vue en coupe partielle d'une pièce de revêtement au repos de la figure 6,
- la figure 8 représente une vue en coupe partielle similaire à la figure 7 lorsque la pièce est soumise à une pression,
- la figure 9 représente schématiquement un assemblage de plusieurs pièces de revêtement selon l'invention,
- la figure 10 représente un exemple de diagramme de flux d'une fonction de configuration de l'assemblage de la figure 9,
- la figure 11 représente une vue sous forme de matrice de l'état d'un ensemble de capteurs de pression,
- la figure 12 représente un exemple de diagramme de flux d'une fonction mise en oeuvre dans l'invention, et
- la figure 13 représente une vue arrachée d'un mode de réalisation d'une pièce de revêtement selon l'invention.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

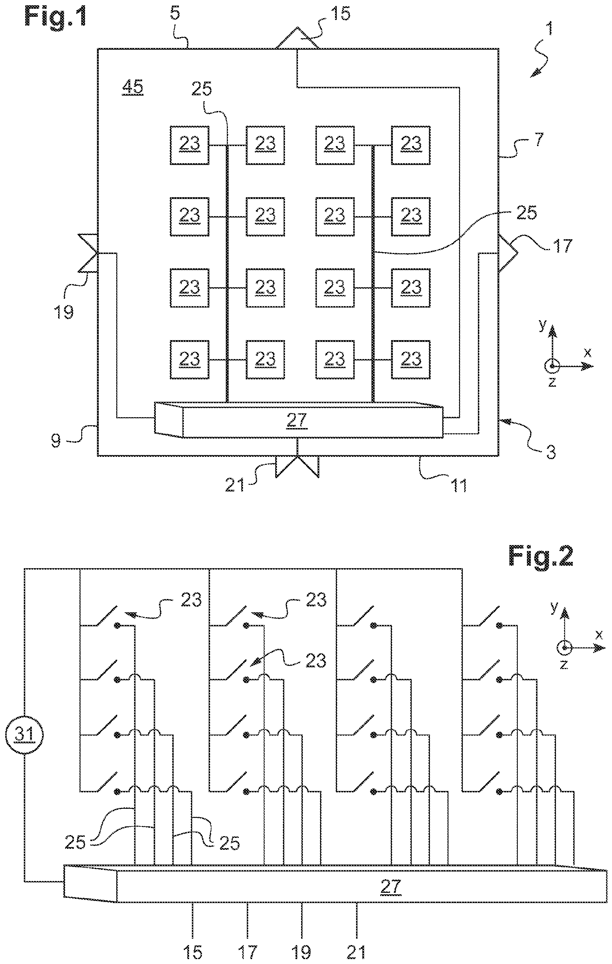

Comme on peut le voir sur la figure 1, une pièce de revêtement 1 comprend un corps 3 délimité par des bords 5, 7, 9, 11, des prises 15, 17, 19, 21, des capteurs de pression 23, des câbles 25 et une unité de traitement 27.

Le terme "pièce" est ici employé dans sons sens général, et désigne un élément destiné à être partie intégrante d'un ensemble plus large, d'un assemblage, à l'image d'une pièce de puzzle. Le terme "pièce" peut notamment désigner un élément de revêtement de sol tel qu'un carreau, une dalle, une lame ou toute autre forme.

La pièce 1 comprend une couche de surface du corps 3 qui n'est pas représentée sur la figure 1 afin de laisser apparaître "l'intérieur" de la pièce de revêtement 1. Dans l'exemple décrit ici, le corps 3 est de forme aplatie et présente deux directions principales et une direction d'épaisseur représentées par le repère (x ; y ; z). Le corps 3 est de forme sensiblement carrée définie par les quatre bords 5, 7, 9 et 11. Pour faciliter la description, on appelle dans ce qui suit "bord nord" le bord 5, "bord est" le bord 7, "bord ouest" le bord 9 et "bord sud" le bord 11. Cette dénomination est indépendante de l'orientation réelle de la pièce de revêtement 1 une fois installée. Les bords 5, 7, 9 et 11 supportent chacun une des prises respectivement 15, 17, 19 et 21. Le corps 3 mesure ici environ 60 cm de côté. En variante, le corps 3 peut mesurer entre 20 cm et 150 cm de côté, par exemple 100 cm, soit une surface comprise entre 400 cm² et 2,25 m².

Dans d'autres variantes, le corps 3 peut avoir une forme autre que carrée, par exemple rectangulaire ou tout autre polygone ou forme à contour fermé adaptée. La pièce de revêtement 1 étant destinée à être assemblée avec d'autres pièces de revêtement similaires, la forme et les dimensions du corps 3 sont choisies pour présenter des correspondances de forme. Autrement dit, le corps 3 forme une maille d'un motif comprenant plusieurs pièces de revêtement assemblées. Les formes et dimensions du corps 3 des pièces de revêtement 1 peuvent être adaptées pour correspondre à des critères esthétiques ou fonctionnels tout en restant combinables entre elles, à l'image d'autres pièces élémentaires de revêtement de sol classiques comme des lames de parquet ou du carrelage.

Comme on le verra par la suite, le corps 3 présente une couche supérieure ou de surface 41 et une couche inférieure ou de base 45.

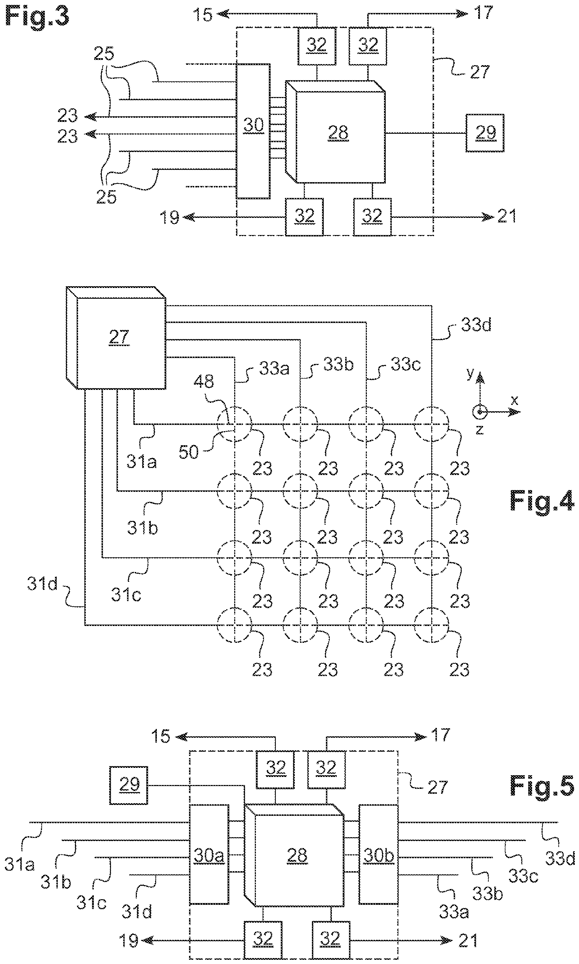

La couche inférieure 45 du corps 3 supporte l'unité de traitement 27 et les capteurs de pression 23. L'unité de traitement 27 sera décrite plus en détail en référence à la figure 3.

Chaque capteur 23 est, ici, électriquement relié à l'unité de traitement 27. Ce lien électrique est assuré par des fils conducteurs 25. Dans l'exemple décrit ici, l'unité de traitement 27 comprend seize entrées, chacune connectée à un fil conducteur 25 lui-même connecté à un des capteurs de pression 23. L'unité de traitement 27 comprend, ici, au moins autant d'entrées que de capteurs de pression 23 qui lui sont associés, auxquelles s'ajoutent quatre entrées/sorties reliées aux prises 15, 17, 19 et 21. En variante, les connexions entre les capteurs de pression 23 et l'unité de traitement 27 sont réalisées au moyen d'un ou plusieurs câbles flexibles plats ou nappes, par exemple vendus par la société Axon sous la référence « Axojump (Marque déposée) - Flat flexible cables ». L'unité de traitement 27 est d'autre part reliée à chacune des prises 15, 17, 19 et 21. Ces liaisons électriques sont ici assurées par des câbles électriques semblables à ceux reliant les capteurs de pression 23 à l'unité de traitement 27. Tout autre moyen adapté de liaison électrique pourra être envisagé par l'homme de l'art.

La figure 2 est un schéma électrique simplifié d'une portion de détection de la pièce 1 comprenant les capteurs de pression 23 et l'unité de traitement 27. Lorsqu'aucune pression n'est détectée, les capteurs de pression 23 sont ici équivalents à des interrupteurs en position ouverte. Lorsqu'une pression est détectée, ils sont équivalents à des interrupteurs en position fermée. La pièce de revêtement 1 comprend une source électrique 31. À l'exception de la figure 2 et pour des raisons de clarté, les sources électriques ne sont pas représentées sur les figures. L'une au moins des prises 15, 17, 19 et 21 renferme ici une source électrique 31. Lorsqu'un capteur de pression 23 subit une pression, la boucle électrique est fermée. L'unité de traitement 27 est apte à déterminer l'état de chacun des capteurs de pression 23 par l'interprétation de variations de courant.

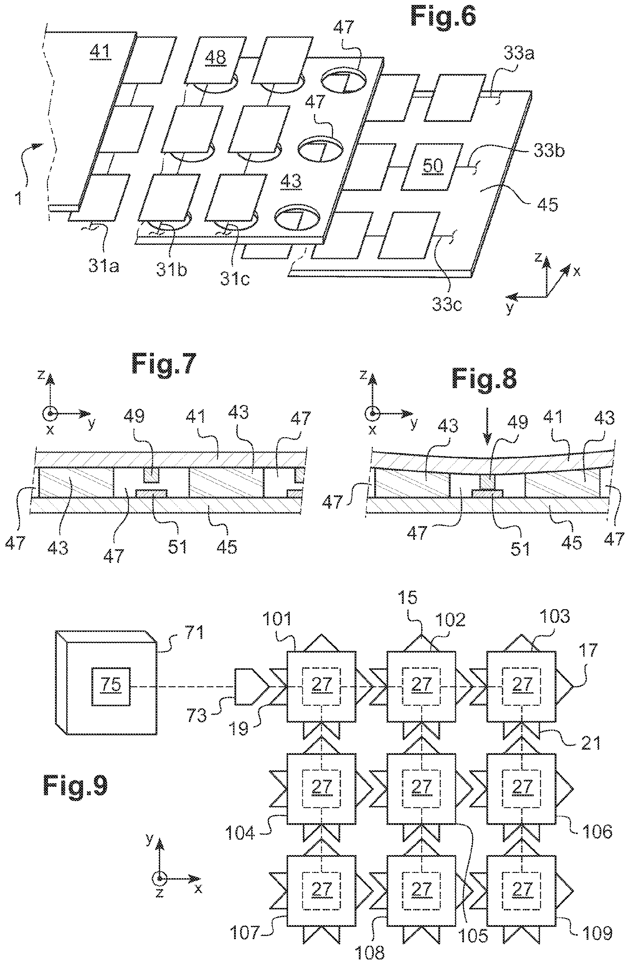

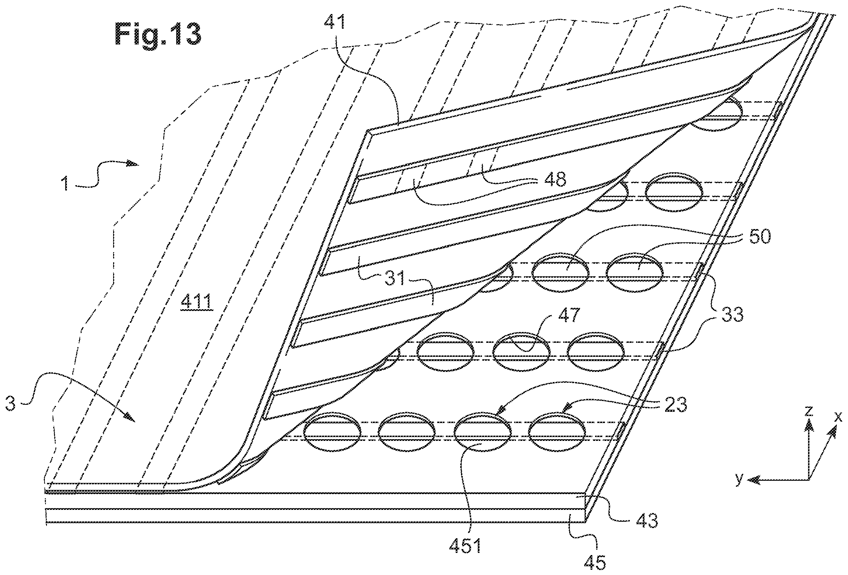

Les capteurs de pression 23 sont répartis sur la surface de la couche inférieure 45 selon une géométrie choisie. Dans l'exemple décrit ici, les capteurs de pression 23 sont au nombre de seize et organisés régulièrement en quatre lignes selon la direction x et quatre colonnes selon la direction y, définissant ainsi une matrice de capteurs. La structure et le fonctionnement individuels d'un mode de réalisation des capteurs de pression 23 seront décrits en référence aux figures 6 à 8. Un autre mode de réalisation sera décrit en référence à la figure 13.

La figure 3 représente l'unité de traitement 27 de la figure 2 et son environnement. L'unité de traitement 27 comprend ici un microcontrôleur 28 connu sous la référence "PIC24FJ64GA306". La référence précitée est adaptée pour une matrice de dix lignes et dix colonnes, soit cent capteurs 23. Le microcontrôleur 28 est choisi notamment pour que ses propriétés soient en adéquation avec la matrice de capteurs 23 à connecter. L'unité de traitement 27 est reliée à une mémoire de masse 29. Le microcontrôleur 28 est relié à un port "capteurs" 30 et à quatre ports "prises" 32. Le port "capteurs" 30 est agencé pour être connecté aux capteurs de pression 23 par l'intermédiaire des fils 25. Les quatre ports "prises" 32 sont chacun agencés pour être connectés à l'une des prises 15, 17, 19 et 21. L'unité de traitement 27 comprend, ici, au moins autant d'entrées que de capteurs de pression 23 qui lui sont associés (soit seize ici), auxquelles s'ajoutent quatre entrées/sorties reliées aux prises 15, 17, 19 et 21. Comme on le verra plus tard, c'est lors d'une opération de configuration de la pièce 1 que chaque entrée/sortie sera définie comme une entrée ou comme une sortie, en fonction de la situation. Le fonctionnement de l'unité de traitement 27 sera décrit plus en détail en référence aux figures 9 à 12.

La figure 4 montre un autre mode de réalisation de la portion de détection de la pièce 1. Les capteurs de pression 23 sont, ici également, organisés en quatre lignes selon la direction x et quatre colonnes selon la direction y. Chaque capteur 23 comprend ici une partie supérieure 48 supportée par la couche de surface 41 et une partie inférieure 50 supportée par la couche inférieure 45. La couche de surface 41 et la couche inférieure 45 ne sont pas représentées. Les parties supérieures 48 des capteurs de pression 23 de chacune des lignes sont reliés à l'unité de traitement 27 par un fil conducteur commun 31a, 31b, 31c, 31d. De manière similaire, les parties inférieures 50 des capteurs de pression 23 de chacune des colonnes sont reliées à l'unité de traitement 27 par un fil conducteur commun 33a, 33b, 33c, 33d. L'unité de traitement 27 peut être par exemple celle décrite dans la suite en référence à la figure 5. Dans l'exemple décrit ici, les parties supérieures 48 sont des portions d'un des fils conducteurs 31a, 31b, 31c, 31d. De même, les parties inférieures 50 sont des portions d'un des fils conducteurs commun 33a, 33b, 33c, 33d. Dans l'exemple décrit ici, les fils des lignes sont disposés selon la direction principale x du corps 3 tandis que les fils des colonnes sont disposés perpendiculairement aux premiers et selon la direction principale y.

Lorsqu'une pression ayant une composante dans la direction de l'épaisseur z de la pièce 1 est appliquée au niveau d'un capteur de pression 23, la partie supérieure 48 et la partie inférieure 50 sont mises en contact. Autrement dit, un contact électrique est établi entre le fil de la ligne 31a, 31b, 31c, 31d concerné et le fil 33a, 33b, 33c, 33d de la colonne concerné, à l'image d'un interrupteur qui se ferme. Dans cette configuration, chaque intersection des fils de ligne 31a, 31b, 31c, 31d et de fil de colonne 33a, 33b, 33c, 33d définit un capteur de pression 23.

En variante, les fils conducteurs peuvent être adaptés au niveau des intersections, et donc des capteurs de pression 23, pour présenter des surfaces de contact électrique plus étendues qu'un croisement de deux fils. Par exemple, les portions de fils concernées peuvent être munies de contacteurs ou de surfaces sensiblement planes et orientées en regard du fil conducteur en vis-à-vis. Dans une autre variante, les fils conducteurs peuvent être remplacés par des bandes souples comprenant un matériau conducteur. En tant que matériau conducteur on peut utiliser par exemple du cuivre ou de l'aluminium. La surface de contact d'une bande permet une meilleure reproductibilité et réduit le risque de non-détection par rapport aux fils. Les capteurs de pressions 23 peuvent être une combinaison de fils et de bandes conductrices. Le mode de réalisation représenté en figure 13 comprend un assemblage de lames conductrices qui sera décrit dans la suite.

En fonctionnement, l'unité de traitement 27 alimente successivement chacune des lignes (ou colonnes) tout en lisant/détectant les variations de courant dans chacune des colonnes (respectivement lignes). Une telle lecture successive des lignes (ou colonnes) permet à l'unité de traitement 27 de déterminer quelles intersections de chacun des fils conducteurs sont en contact électrique et donc l'état individuel de chacun des capteurs de pression 23. Le nombre de fils conducteurs entre les capteurs de pression 23 et l'unité de traitement 27 et le nombre d'entrées de l'unité de traitement 27 dédiées aux capteurs de pression 23 sont ainsi réduits (huit au lieu de seize) dans l'exemple décrit ici. Le nombre de fils et d'entrées de la portion de détection pour l'unité de traitement 27 est égal à la somme des lignes et des colonnes de la matrice de capteurs, ici 4 + 4. Les signaux issus des capteurs de pression 23 sont multiplexés.

La figure 5 représente l'unité de traitement 27 de la figure 4 et son environnement. L'unité de traitement 27 comprend ici un microcontrôleur 28 connu sous la référence "PIC24FJ64GA306". L'unité de traitement 27 est reliée à une mémoire de masse 29. Le microcontrôleur 28 est relié à un port "capteurs-lignes" 30a, à un port "capteurs-colonnes" 30b et à quatre ports "prises" 32. Les ports "capteurs-lignes" 30a et "capteurs-colonnes" 30b sont agencés pour être connectés aux capteurs de pression 23 par l'intermédiaire de fils 31a, 31b, 31c et 31d, respectivement 33a, 33b, 33c et 33d qui seront décrits plus en détail par la suite. Les quatre ports "prises" 32 sont chacun agencés pour être connectés à l'une des prises 15, 17, 19 et 21. L'unité de traitement 27 comprend, ici, moins d'entrées (huit) que l'unité de traitement décrite en référence à la figure 3. Quatre entrées/sorties reliées aux prises 15, 17, 19 et 21 s'ajoutent à ces huit entrées. Comme on le verra plus tard, c'est lors d'une opération de configuration de la pièce 1 que chaque entrée/sortie sera définie comme une entrée ou comme une sortie, en fonction de la situation. Le fonctionnement de l'unité de traitement 27 sera décrit plus en détail en référence aux figures 9 à 12.

Comme on peut le voir sur la figure 6, le corps 3 de la pièce de revêtement 1 comprend la couche de surface 41, une couche intermédiaire 43 et la couche inférieure 45. L'agencement de la pièce 1 est, ici, une variante du mode de réalisation de la figure 4 permettant le multiplexage. La partie supérieure 48 et la partie inférieure 50 de chaque capteur de pression 23 comprend une plaque en matériau conducteur. Ces plaques sont reliées :
- par lignes pour les parties supérieures 48 par des fils conducteurs 31a, 31b et 31c, et
- par colonnes pour les parties inférieures 50 par des fils conducteurs 33a, 33b et 33c.

La couche intermédiaire 43 est située sous la couche de surface 41, au contact des parties supérieures 48 et sur la couche inférieure 45, au contact des parties inférieures 50. Dans l'exemple décrit ici, la couche intermédiaire 43 est réalisée en un matériau isolant électriquement, par exemple une couche en matériau plastique isolant. Ici, on utilise du polyester perforé. En variante, un film à base de polypropylène (PP), par exemple pour ses propriétés ignifuges, ou de polyimide est utilisé. La couche de surface 41 et/ou la couche inférieure 45 peuvent également être réalisées à partir d'un film de polyester, de polypropylène (PP), ou de polyimide. Selon les réalisations, la couche de surface 41, la couche intermédiaire 43 et la couche inférieure 45 peuvent être réalisées dans le même matériau ou dans des matériaux distincts.

La couche intermédiaire 43 comprend une multiplicité d'évidements 47 ou trous qui laissent les parties supérieures 48 en regard des parties inférieures 50.

Les évidements 47 sont des trous traversants dans la direction de l'épaisseur z, pratiqués régulièrement dans la couche intermédiaire 43. Dans l'exemple décrit ici, ces évidements 47 ont une forme circulaire ayant un rayon de 1 cm. Dans d'autres modes de réalisation, la forme de ces évidements 47 pourra varier, et être par exemple un rectangle, un losange ou tout autre polygone adapté, ou encore un contour fermé quelconque, notamment de révolution. L'évidement 47 présente une surface choisie, par exemple comprise entre moins de 1 cm² et 9 cm², pour une épaisseur de la couche intermédiaire 43 comprise entre environ 75 µm et 12 mm, par exemple 100, 200 ou 300 µm. Dans l'exemple décrit ici, les évidements 47 sont de forme circulaire, présentant un diamètre d'environ 10 mm, soit une surface d'environ 0,8 cm² pour une épaisseur de couche intermédiaire 43 d'environ 100 µm.

De fait, les évidements 47 sont prévus pour permettre la déformation de la couche de surface 41 à travers ceux-ci, afin que les parties supérieures 48 viennent à proximité et/ou au contact (avoisine) de la couche inférieure 45.

Dans l'exemple décrit ici, la couche de surface 41 est superposée sur la couche intermédiaire 43, qui est elle-même superposée sur la couche inférieure 45, dans cet ordre. Autrement dit, la couche intermédiaire 43 est prise en sandwich entre la couche de surface 41 en haut et la couche inférieure 45 en bas.

Les figures 7 et 8 représentent des vues en coupe d'une pièce de revêtement 1 respectivement au repos et en réponse à une pression possédant une composante verticale. L'agencement de la pièce 1 est, ici, une variante selon le mode de réalisation de la figure 2 dans laquelle chaque capteur de pression 23 fonctionne comme un interrupteur d'une boucle distincte de celle des autres capteurs 23.

La couche de surface 41 comprend un conducteur électrique 49 qui s'étend en plusieurs lignes parallèles selon la direction x de la pièce de revêtement 1.

Dans l'exemple décrit ici, le conducteur 49 est un unique fil électrique présentant un diamètre d'environ 1 mm, qui est relié à une alimentation électrique non représentée. Le fil conducteur 49 repose dans l'exemple décrit ici sur une face inférieure de la couche de surface 41, ce qui l'isole électriquement. Le fil conducteur 49 peut être isolé en outre par une gaine, ou pas. Le diamètre de ce fil électrique 49 peut varier, en fonction des besoins en courant et des options d'alimentation envisagées. Ce fil électrique 49 unique peut être remplacé par une pluralité de fils isolés électriquement les uns et les autres et chacun relié à une alimentation électrique indépendante. Ce peut être aussi un système conducteur, par exemple de type circuit imprimé mono ou multi-conducteur, ou équivalent. Ce peut également être une couche conductrice qui revêt la face inférieure de la couche de surface 41.

Le fil conducteur 49 a pour fonction de se déformer sous une pression selon une direction sensiblement verticale z pour établir un contact électrique permettant de détecter cette pression.

La couche intermédiaire 43 est similaire à celle décrite en référence à la figure 6. Les évidements 47 sont, ici, prévus pour permettre la déformation de la couche de surface 41 à travers ceux-ci, afin que le fil conducteur 49 vienne à proximité et/ou au contact ou avoisine de la couche inférieure 45. La portion du fil conducteur 49 qui vient à proximité et/ou au contact de la couche inférieure 45 forme un segment conducteur.

La couche inférieure 45 présente une pluralité de contacts électriques 51, qui sont chacun relié par un fil à l'unité de traitement 27 conformément au mode de réalisation de la figure 2. Dans ce mode de réalisation, un capteur de pression 23 comprend une portion de fil conducteur 49, le contact électrique 51 disposé en vis-à-vis et l'évidement 47 qui les loge.

Dans l'exemple décrit ici, les contacts électriques 51 de la couche inférieure 45 sont choisis pour présenter une surface de contact 3 à 5 fois plus importante que la surface de contact d'un conducteur électrique 49 de la couche de surface 41. En effet, cela facilite le contact avec ce dernier lors d'une déformation de la couche de surface 41 suite à une pression, et évite les erreurs de détection. Cependant, dans diverses variantes, la surface du contact électrique 51 pourra être choisie identique à celle du conducteur électrique 49, ou inférieure.

Dans un autre mode de réalisation, les capteurs 23 peuvent encore être des boutons poussoirs.

La pièce de revêtement 1 est donc prévue pour être installée avec la couche inférieure 45 au contact d'un sol existant, et avec la couche de surface 41 comme surface de contact pour la marche. À cet effet, la couche de surface 41 peut avantageusement, du moins sa surface supérieure extérieure, être du linoléum, du plastique, de la moquette, ou encore tout type de matériau et de structure de sol tel que défini par des normes sanitaires.

En variante, la surface supérieure de la couche de surface 41 peut être prévue pour accueillir une couche supplémentaire d'un revêtement de sol classique et éventuellement être munie d'adhésif facilitant la fixation dudit revêtement classique. Les pièces de revêtement 1 sont alors considérées comme des éléments d'une sous-couche de revêtement de sol tandis que la couche supplémentaire forme la couche de surface, pour la marche. Dans ce cas, la flexibilité de la couche de surface 41, la sensibilité des capteurs de pression 23 et/ou ledit revêtement supplémentaire classique sont adaptés de sorte à ne pas altérer ou neutraliser le fonctionnement de la pièce de revêtement 1. Par exemple, le revêtement supplémentaire est choisi suffisamment déformable pour qu'en cas de pression verticale, la couche de surface 41 s'enfonce dans les évidements 47 à l'endroit où la pression est appliquée, comme décrit précédemment. Il est souhaitable d'éviter des revêtements supplémentaires trop rigides tels que du carrelage qui pourraient répartir l'application des forces en jeu sur la (les) pièce(s) et fausser les mesures.

Avantageusement, la couche de surface 41 peut être choisie plus déformable que la couche intermédiaire 43, qui pourra par exemple présenter une résistance à la pression d'environ 15 kg/m² à 25 kg/m². Ainsi, la couche de surface 41 peut se déformer plus facilement à l'intérieur des évidements 47 sous l'effet d'une pression, ce qui permet d'augmenter la sensibilité de détection.

De la même manière, la couche inférieure 45 est adaptée pour servir de liaison à un sol. Elle peut être par exemple en caoutchouc, présenter des propriétés antidérapantes, être une matière adaptée au collage et/ou comprendre d'autres moyens de fixation.

Au-delà de leur superposition, les couches 41, 43 et 45 sont spécifiquement agencées de sorte que le conducteur 49 soit disposé en regard des évidements 47 ou du moins de leur plus grande majorité, et de sorte que les contacts électriques 51 soient eux-mêmes en regard de ces évidements 47 ou de leur plus grande majorité.

Le corps 3 de la pièce de revêtement 1 comprend la couche inférieure 45 et la couche de surface 41. Ces deux couches 41, 45 sont assemblées à leurs bords mutuels pour former les bords 5, 7, 9 et 11 décrits en référence à la figure 1. L'assemblage est, ici, réalisé par soudage au voisinage de la périphérie. En variante, les deux couches 41, 45 sont reliées par tissage, collage, sertissage et/ou tout autre moyen adapté. Dans une autre variante, la couche inférieure 45 et la couche de surface 41 sont formées d'une pièce intégrale repliée sur elle-même. L'un des bords 5, 7, 9 et 11 étant alors formé par le pli tandis que les trois autres bords sont formés par l'assemblage mutuel des bords libres. D'une manière générale, le corps 3 forme une enceinte ou une enveloppe protectrice et/ou sensiblement étanche pour les composants intérieurs de la pièce de revêtement 1 par rapport à l'environnement extérieur. En particulier, les composants électroniques sont protégés des liquides pouvant être en contact avec la pièce 1.

Comme cela est représenté sur la figure 8, lorsqu'une pression (représentée sur cette figure par une flèche), par exemple la force exercée par le poids d'une personne, est exercée sur la couche de surface 41, celle-ci se déforme et vient remplir les évidements 47 au niveau de l'endroit où cette pression est exercée. Le conducteur 49 vient alors au contact des contacts électriques 51 dans les évidements 47 concernés.

Dans l'exemple décrit ici, les évidements 47 sont espacés selon l'une et l'autre des directions principales x et y de la pièce de revêtement 1, centre à centre, d'une distance d'environ 7,5 cm, et si l'on considère une pièce de revêtement 1 présentant une surface de 1,6 m par 2,1 m, on obtient donc 252 capteurs de pression 23 formés par les triplets conducteur 49, évidement 47, contact 51. Avantageusement, l'espacement entre les évidements 47 peut être compris entre 5 cm et 20 cm.

Dans le mode de réalisation représenté en figure 13, les éléments fonctionnant de manière similaire à ceux des modes de réalisation précédemment décrits sont numérotés de manière identique. La couche intermédiaire 43 présente une épaisseur et une composition choisies pour leurs propriétés élastiques ou rigides. La couche de surface 41 et la couche inférieure 45 sont chacune réalisée à base d'un film, respectivement 411 et 451, à base de polyester, polyéthylène ou polyimide. Les films 411 et 451 présentent des épaisseurs comprises entre 1 et 5 mm, par exemple 3 mm.

Les faces des films 411 et 451 en regard, c'est-à-dire orientées vers l'intérieur du corps 3, portent des lames conductrices 31, respectivement 33. Les lames conductrices 31 portées par la surface du film 411 de la couche de surface 41 dirigée vers l'intérieur forment un jeu de lames conductrices 31. Les lames conductrices 33 portées par la surface intérieure du film 451 de la couche inférieure 45 dirigée vers l'intérieur forment un autre jeu de lames conductrices 33.

Les lames conductrices 31, 33 sont réalisées sous la forme de pistes en cuivre, en aluminium ou en tout autre matériau conducteur adapté, par dépôt ou par vernis, en vue de former des câbles plats, c'est-à-dire présentant une épaisseur faible par rapport à leur surface.

En variante, les lames conductrices 31, 33 de chacun des jeux sont fixées au film 411, respectivement 451, par exemple par collage ou application d'une résine réactivable à chaud. Les lames conductrices 31, 33 sont alors appliquées directement contre les films 411, respectivement 451, par pression entre deux rouleaux et sous chauffage. On parle alors "d'enduction" : le matériau du film est lui-même réactivable à chaud. On utilise par exemple comme matériau un polyester-imide, qui présente de bonnes propriétés d'adhésion avec le cuivre.

Les lames conductrices 31, 33 présentent ici des épaisseurs comprises entre 30 et 200 µm, par exemple 50 µm. Les lames conductrices 31, 33 sont de formes sensiblement similaires les unes aux autres. La longueur et la largeur des lames conductrices 31, 33 sont adaptées aux dimensions de la pièce 1. Dans l'exemple, les lames conductrices 31, 33 présentent des longueurs légèrement inférieures à celle de la pièce 1, c'est-à-dire ici inférieures à 50 cm. Les lames conductrices 31, 33 présentent des largeurs comprises entre 2 et 20 mm, par exemple 6 mm.

Les lames conductrices 31, 33 sont disposées à plat contre les surfaces intérieures des films 411, respectivement 451. Les lames conductrices 31 du premier jeu sont parallèles entre elles. Les lames conductrices 33 du second jeu sont parallèles entre elles. Les lames conductrices 31, 33 s'étendent selon des directions (x, respectivement y) comprises dans des plans parallèles au plan principal de la pièce 1. Les lames conductrices 31 du premier jeu s'étendent selon une direction (la direction x) sensiblement perpendiculaire à celle (la direction y) des lames conductrices 33 du second jeu. Les lames conductrices 31, 33 sont espacées les unes des autres d'un pas sensiblement constant. Les deux jeux de lames conductrices 31, 33 superposés forment alors un quadrillage sensiblement homogène et régulier. Les lames conductrices 31 du premier jeu forment une série de lignes selon la direction x. Les lames conductrices 33 du second jeu forment une série de colonnes selon la direction y.

Les lames conductrices 31 du premier jeu croisent les lames conductrices 33 du second jeu au niveau des évidements 47. Par se croiser, on entend ici un croisement vu selon une direction perpendiculaire au plan principal de la pièce 1 (la direction z), sans que les lames conductrices 31, 33 soient en contact lorsque la pièce 1 n'est soumise à aucune contrainte. Les lames conductrices 31, 33 de chacun des jeux restent maintenues à distance selon la direction z de l'épaisseur par la couche intermédiaire 43.

Lorsqu'une pression ayant une composante dans la direction z est appliquée au niveau d'un croisement entre le premier jeu de lames et le deuxième jeu de lames, la portion 48 de la lame conductrice 31 et la portion 50 de la lame conductrice 33 correspondantes sont mises en contact. Un contact électrique est établi entre la lame conductrice 31 concernée et la lame conductrice 33 correspondantes, à l'image d'un interrupteur qui se ferme. Dans cette configuration, chaque intersection des lames conductrices 31 et 33 définit un capteur de pression 23.

Des portions 48 des lames conductrices 31 sont en regard de portions 50 des lames conductrices 33 au travers des évidements 47. Chaque couple de portions 48, 50 de lames conductrices 31, 33 en regard au travers d'un évidement 47 commun forme un capteur de pression 23. L'ensemble des couples de portions 48 ; 51 de lames conductrices 31 ; 33 fixées sur les surfaces intérieures des films 411 ; 451 des couches 41 ; 45 forme une matrice de capteurs de pression 23.

L'unité de traitement 27 comprend un circuit imprimé, par exemple un circuit intégré propre à une application (ou ASIC pour "Application-Specific Integrated Circuit" en anglais), un circuit logique programmable (par exemple connu sous le sigle FPGA pour "Field-Programmable Gate Array" en anglais) ou encore un système intégré sur puce (ou SoC pour "System on Chip" en anglais).

Dans un mode de réalisation préféré, le corps 3 est de forme générale rectangulaire ou carrée. L'unité de traitement 27 est disposée dans un coin du corps 3. Ainsi, les bords opposés à ce coin sont peuvent être découpés préalablement à l'installation de la pièce de revêtement 1. Cela permet d'adapter la forme de la pièce de revêtement 1 lors de la pose en fonction des formes et dimensions du sol à couvrir. La possibilité de découpe doit être comprise ici comme une coupe au moyen de ciseaux ou d'outils classiques tels qu'un couteau à moquette généralement à disposition d'un opérateur habitué à poser des revêtements de sols. En effet, lors de l'installation dans un coin d'une salle, les deux prises prévues sur les bords opposés au coin de la pièce dans lequel est disposée l'unité de traitement 27 ne sont plus utiles. Aussi, la portion de la pièce de revêtement 1 correspondante peut être sectionnée pour accommoder la forme de la salle dans laquelle le revêtement est posé. Cela permet donc une grande souplesse d'installation, et la pièce de revêtement 1 peut ainsi être fabriquée selon un gabarit standard tout en étant adaptable à des formes complexes ou non usuelles.

On a décrit jusqu'ici des modes de réalisation d'une pièce de revêtement 1. Cependant, et comme évoqué plus haut, les pièces de revêtement de l'invention ont pour vocation d'être assemblées avec des pièces de revêtement similaires pour recouvrir un sol existant. La pièce de revêtement 1 est donc agencée pour être connectée à d'autres pièces de revêtement similaires, ou du moins compatibles. Dans cette optique, il est avantageux de fournir un ensemble ou lot de plusieurs pièces de revêtement 1, par exemple sous la forme d'un kit d'installation.

La figure 9 montre un exemple d'un tel assemblage comprenant neuf pièces de revêtement 101 à 109 mutuellement organisées en trois lignes et trois colonnes.

Les prises 15, 17, 19 et 21 des pièces de revêtement 101 à 109 sont agencées pour pouvoir être branchées avec l'une au moins des prises 15, 17, 19 et 21 d'une pièce de revêtement juxtaposée. Dans l'exemple décrit ici, les prises nord 15 et est 17 de chacune des pièces de revêtement 101 à 109 sont de forme mâle. De manière complémentaire, les prises ouest 19 et sud 21 de chacune des pièces de revêtement 101 à 109 sont de forme femelle. De manière générale, deux prises destinées à être connectées ensemble sont de formes complémentaires.

Dans un état assemblé, tel que représenté sur la figure 9, les pièces de revêtement 101 à 109 de sol sont interconnectées. La transmission des informations entre chaque unité de traitement 27 de chaque pièce de revêtement 101 à 109 est rendu possible par les connexions mutuelles des prises 15, 17, 19 et 21.

Un tel agencement des prises permet en outre de remplir la fonction de détrompeur. Dans cette configuration, si la couche inférieure 45 de chaque pièce de revêtement 101 à 109 est orientée vers le sol et la couche de surface 41 orientée vers le haut, alors chaque pièce de revêtement 101 à 109 est nécessairement orientée de manière semblable aux autres pour pouvoir s'y connecter. Des différences de textures, matériaux et/ou couleurs entre les surfaces visibles de la couche de surface 41 et de la couche inférieure 45 permet de réduire le risque d'inversion des deux faces principales des pièces 101 à 109.

En variante, la couche de surface 41 et la couche inférieure 45 peuvent être semblables lorsque les pièces de revêtement sont prévues pour être appliquées au sol indépendamment de l'orientation des surfaces. Les pièces de revêtement sont alors réversibles.

En variante, les prises peuvent être toutes du même type, par exemple toutes mâles ou toutes femelles. Dans ce cas, il est prévu un élément distinct tel qu'un cavalier à interposer entre deux prises à connecter, le cavalier étant par exemple de type femelle-femelle ou mâle-mâle. Cette variante présente les avantages de ne nécessiter qu'un seul type de prise et réduit les coûts de fabrication. D'autre part, l'orientation des pièces de revêtement peut alors être libre, ce qui facilite le travail de l'opérateur lors de l'installation du revêtement. Les pièces en tant que telles sont alors dépourvues de contrainte d'orientation à l'installation. L'orientation d'une telle pièce n'est définie qu'a posteriori durant la configuration et relativement à sa position par rapport à d'autres pièces comme cela sera décrit dans la suite.

Dans une autre variante, les prises peuvent être à superposition au moins partielle. En d'autres termes, au cours de l'installation d'une pièce de revêtement au voisinage immédiat d'une pièce de revêtement déjà installée, une prise de la seconde pièce recouvre au moins en partie une prise de la première pièce. La connexion est alors réalisée selon une direction sensiblement perpendiculaire au plan du corps. Ceci permet d'effectuer l'opération de branchement sensiblement simultanément à la pose par un mouvement sensiblement vertical. Cette variante est combinable avec la variante à cavalier dans le cas où chacune des prises est agencée pour fonctionner aussi bien en tant que prise recouverte qu'en tant que prise recouvrante.

Dans d'autres variantes, les prises pourront être des dispositifs sans contact, par exemple des éléments communicants de type capacitif ou inductif. Dans ce cas, les prises de deux dalles voisines peuvent être disposées dans le corps respectif de chaque dalle et communiquer avec une prise associée au travers des enveloppes formées par les corps de ces dalles, sans être accessibles depuis l'extérieur. Ceci facilite l'isolation de l'intérieur du corps par rapport à l'environnement extérieur. Le risque d'infiltration de fluide par une prise dans la pièce de revêtement et de détériorations est limité.

Le montage, en état de fonctionnement, comprend, outre les pièces de revêtement 101 à 109, une unité centrale 71 ou analyseur pour la détection de chutes.

L'unité centrale 71 comprend une prise 73. La prise 73 est agencée pour être branchée à au moins une pièce de revêtement 101 à 109, par exemple par l'intermédiaire d'une prise 15, 17, 19 et 21 de ladite pièce. Ici, la prise 73 mâle de l'unité centrale 71 est connectée à la prise ouest 19 femelle de la pièce de revêtement 101 elle-même située dans le coin nord-ouest de l'assemblage.

L'unité centrale 71 comprend un processeur 75 relié à la prise 73.

Une fois qu'un ensemble de pièces de revêtement 101 à 109 est installé au sol et interconnecté, le processeur 75 est relié à une unité de traitement 27 de l'une des pièces de revêtement 101 à 109 et mis sous tension. À l'allumage, ou suite à un redémarrage ou à une remise à zéro, le processeur 75 exécute une opération de configuration.

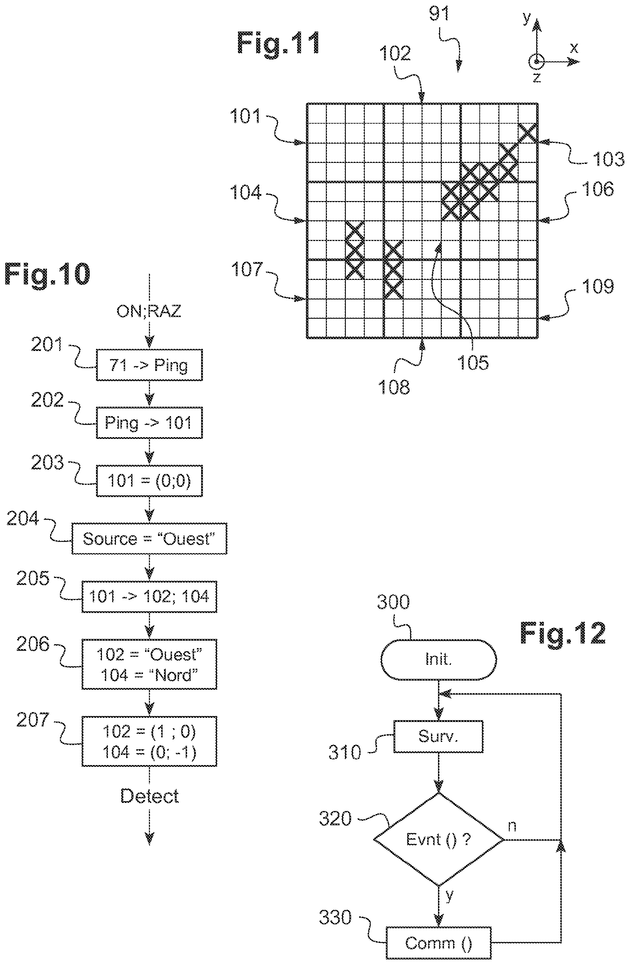

La figure 10 représente un diagramme de flux que le processeur 75 et les unités de traitement 27 peuvent mettre en oeuvre à cet effet. Dans une opération 201, le processeur 75 émet un signal de configuration, ou "ping", par l'intermédiaire d'une sortie de l'unité centrale 71, et ici la prise 73.

Dans une opération 202, le signal de configuration est reçu par une première pièce de revêtement à laquelle est branchée la prise 73, soit la pièce 101 dans le cas de l'agencement de la figure 9. Le signal est reçu par l'unité de traitement 27 de la pièce 101 par l'intermédiaire d'une des prises, ici la prise ouest 19. L'unité de traitement 27 identifie le signal de configuration ainsi que la prise source, ici la prise ouest 19, par laquelle le signal a été reçu.

Le signal de configuration comprend l'identifiant de localisation d'une première pièce, unique, à laquelle est connectée l'unité centrale 71, ici la pièce de revêtement 101. Cet identifiant est ici un couple de coordonnées dans un plan correspondant au sol de type (x ; y) et fixant une origine (0 ; 0). Comme cela sera décrit par la suite, la fixation de cette origine permet de définir la propagation des informations au sein de l'assemblage de pièces 101 à 109 et de l'unité centrale 71. Cet identifiant de localisation devient, ici, à la fois une information d'identification de la pièce 101 et une information de localisation de la pièce 101 relativement à l'assemblage.

Dans une opération 203, l'unité de traitement 27 de la pièce 101 interprète le signal de configuration comme une désignation de la pièce de revêtement 101 en tant que première pièce de l'assemblage. L'unité de traitement 27 stocke dans sa mémoire l'identifiant de localisation (0 ; 0) de la pièce de revêtement 101.

Dans une opération 204, qui peut être réalisée avant, pendant ou après l'opération 203, l'unité de traitement 27 interprète la source du signal de configuration comme une désignation de la prise source, ici la prise ouest 19. Par exemple, l'unité de traitement 27 stocke dans sa mémoire un identifiant de prise source ou d'entrée. Cet identifiant est ici "ouest". La fixation de cette orientation permet également de définir la propagation des informations au sein de l'assemblage de pièces 101 à 109 et de l'unité centrale 71. La prise ouest 19 est alors définie comme une entrée.

En variante, le signal de configuration émis par l'unité centrale 71 est vierge, c'est-à-dire qu'il ne comprend aucun identifiant de localisation. Dans ce cas, chaque unité de traitement 27 est agencée pour attribuer un identifiant de localisation fixant l'origine (0 ; 0) à la pièce de revêtement à laquelle il appartient en réponse à la réception du signal de configuration vierge.

Dans une opération 205, l'unité de traitement 27 de la première pièce, ici 101, émet des signaux de configuration modifiés. Chacun de ces signaux de configuration modifiés est envoyé vers l'une des trois autres prises 15, 17, 21 qui deviennent alors des sorties. Le signal de configuration modifié comprend, ici, l'identifiant de localisation de la pièce de revêtement émettrice 101, l'identifiant de localisation de la pièce de revêtement réceptrice 102, respectivement 104, et l'identifiant de la prise source, ici "ouest" pour la pièce 102 et "nord" pour la pièce 104. L'unité de traitement 27 de la pièce 101 détermine l'identifiant de la pièce 102, respectivement 104 en fonction de l'identifiant de la pièce de revêtement 101 émettrice et de la prise par laquelle le signal est émis. Par exemple, l'identifiant à transmettre par la prise est 17 de la pièce 101 correspond à celui de la pièce 101 dont la coordonnée relative à la direction est-ouest (x) est incrémentée de 1, soit (1 ; 0). De manière analogue, l'identifiant à transmettre par la prise sud 21 de la pièce 101 correspond à celui de la pièce 101 dont la coordonnée relative à la direction nord-sud (y) est décrémentée de 1, soit (0 ; -1). Les informations d'identification à transmettre sont sélectionnées ici de manière itérative par les unités de traitement 27.

Dans une opération 206, chaque signal de configuration modifié est reçu par chacune des pièces de revêtement 102, 104, dites réceptrices, connectées à l'une des trois prises sorties 15, 17, 21 de la pièce émettrice 101. Le signal est reçu par l'unité de traitement 27 de chacune desdites pièces 102, 104 par l'intermédiaire d'une des prises, ici la prise ouest 19 de la pièce 102 et la prise nord 15 de la pièce 104. Chacune des unités de traitement 27 identifie le signal de configuration modifié ainsi que la prise, par laquelle le signal a été reçu. L'unité de traitement 27 stocke en mémoire un identifiant de la prise source, ici la prise ouest 19 pour la pièce 102, respectivement la prise nord 15 pour la pièce 104. Cette prise est alors définie comme la prise entrée.

Dans une opération 207, chacune des unités de traitement 27 interprète le signal de configuration modifié. Ici, chaque unité de traitement 27 stocke en mémoire l'identifiant de localisation (1 ; 0), respectivement (0 ; -1), de la pièce de revêtement 102, respectivement 104, à laquelle il appartient et l'identifiant de localisation (0 ; 0) de la pièce de revêtement source 101 compris dans le signal reçu de la pièce émettrice 101. L'unité de traitement 27 calcule les informations de localisation de la pièce en fonction d'autres informations de localisation. Ce calcul est itératif.

Ensuite, chacune des pièces 102, 104 réceptrices transmet à son tour le signal par chacune des trois autres prises non définies comme entrées et donc définies comme sorties. Cette opération est similaire à celle de l'opération 205, les pièces 102, 104 réceptrices devenant à leur tour émettrices du signal de configuration modifié pour les autres pièces 103, 105, 107 juxtaposées. Par exemple, l'unité de traitement 27 de la pièce de revêtement 102 détermine par comparaison des identifiants de localisation des pièces 101 et 102 que les pièces 101 et 102 sont alignées selon la direction correspondant à la coordonnée x. En effet, la coordonnée x a été incrémentée entre les pièces de revêtement 101 et 102. Ceci permet à l'unité de traitement 27 de la pièce de revêtement 102 de calculer de quelle manière incrémenter (ou décrémenter) son propre identifiant de localisation (1 ; 0) pour calculer les identifiants de localisation à transmettre à chacune des prises sorties, ici : (2 ; 0) par la prise est 17 à destination de la pièce de revêtement 103 et (1 ; -1) par la prise sud 21 à destination de la pièce de revêtement 105. Le signal de configuration est ainsi transmis de proche en proche pour configurer successivement chacune des pièces connectées de l'assemblage.

Les unités de traitement 27 sont configurées pour ignorer les signaux de configurations une fois que l'identifiant de localisation et la prise "entrée" sont fixés et stockés dans la mémoire de l'unité de traitement 27, de sorte à ne définir qu'une seule et unique prise "entrée". Par exemple, pour le cas de la pièce 105, l'unité de traitement 27 de cette pièce traitera seulement le signal reçu de la pièce 102 ou seulement le signal reçu de la pièce 104. Cette caractéristique est réversible et peut être annulée par une commande de remise à zéro. Par exemple, un signal spécifique émis par l'unité centrale 71 peut effacer les informations stockées dans la mémoire des unités de traitement 27.

En variante, la transmission du signal de configuration peut être limitée à certaines prises sorties seulement afin de limiter l'échange d'information.

Dans une variante du processus de configuration, l'état d'activation des capteurs de pression 23 de chacune des pièces de revêtement 101 à 109 est relevé puis fixé comme état de référence. Si durant cette étape de calibrage, certains capteurs de pression 23 sont détectés comme activés (une pression est détectée), alors le processeur 75 ignore les informations d'état relatives à ces capteurs 23 dans le futur et en cours de fonctionnement. Cette calibration est réalisée lorsqu'aucune personne ne se trouve dans la pièce mais que le mobilier habituel est présent. Ainsi, des capteurs de pression 23 détectant une pression, due par exemple au poids d'un meuble, peuvent être neutralisés ou du moins ignorés lors des calculs décrits par la suite. De cette manière, la présence de mobilier préserve le fonctionnement de la mesure et donc la véracité des alertes.

Dans un autre mode de réalisation, la calibration peut être générée à intervalle régulier par l'envoi d'un signal de configuration par le processeur 75, par exemple toute les six heures. Dans ce mode de réalisation, la défaillance d'une connexion entre deux prises de deux pièces juxtaposées peut être résolue automatiquement. Le signal de configuration pourra être transmis de proche en proche en empruntant un cheminement contournant la connexion défectueuse. Le parcours ou schéma de transit d'information ou routage adapté est ainsi défini tant que les connexions actives sont suffisantes. Ce mode de réalisation est adapté aux pièces de revêtements comprenant plus de prises que strictement nécessaires, par exemple quatre. Dans ce mode de réalisation, la neutralisation de certains capteurs 23 peut être prévue lorsqu'une pression est détectée de manière systématique lors de calibrations successives.

Une fois qu'une pièce est configurée, c'est-à-dire que ses identifiants de localisation et l'identifiant de la prise source ont été enregistrés dans la mémoire de son unité de traitement 27, cette pièce passe de l'état de configuration à un état de détection.

En état de détection, chaque unité de traitement 27 transmet un message d'état. Ce message d'état comprend l'état "pression détectée" ou "pas de pression détectée" de certains au moins des capteurs de pression 23 reliés à l'unité de traitement 27. Cette transmission est effectuée par l'intermédiaire de la prise entrée dont l'identifiant est stocké dans la mémoire de l'unité de traitement 27. En état de détection, les prises "entrées" de la phase de configuration sont utilisées comme des sorties et vice versa. Le message d'état comprend en outre un identifiant de localisation de chacun desdits capteurs 23 relativement à la pièce en question, l'identifiant de localisation de la pièce établi durant le processus de configuration et un marqueur de temps. Le couple identifiant de localisation de la pièce / identifiant de localisation du capteur dans la pièce permet d'identifier et de localiser chacun des capteurs 23 de l'assemblage. Chaque unité de traitement 27 associe ces informations pour former le message. Autrement dit, outre l'état d'activation et l'identification de chacun des capteurs de pression 23 qui lui sont reliés, l'unité de traitement 27 applique un marqueur contenant des informations de localisation de la pièce de manière à distinguer les informations de deux capteurs 23 homologues de deux pièces distinctes. Ceci permettra a posteriori une reconnaissance de la pièce de revêtement portant lesdits capteurs de pression 23.

Dans l'exemple décrit ici, l'état de chacun des capteurs 23 ou seulement de ceux dont l'état a changé, est compris dans un message transmis à intervalles de temps prédéfinis.

En variante, dès qu'un changement d'état d'un capteur 23 est détecté, l'état de l'ensemble des capteurs 23 est compris dans le message.

Dans une autre variante, le message n'est transmis que lorsque un changement d'état d'au moins un des capteurs 23 est détecté et seuls les états des capteurs 23 pour lesquels un changement a été détecté sont inclus dans ledit message. On parle alors "d'attente active" ou de "polling".

D'autre part, en état de détection, chaque unité de traitement 27 recevant un message d'état par l'une de ses prises "sorties" transmet ce message par sa prise "entrée". Ainsi, les messages d'état sont transmis de proche en proche dans le sens inverse de celui pris par le signal lors de l'opération de configuration pour remonter jusqu'au processeur 75. L'unité de traitement 27 émet les informations associées et les informations reçues à des intervalles de temps prédéfinis. Un tel agencement de l'unité de traitement 27 permet de regrouper des informations d'origines diverses pour les transmettre par un nombre limité de canaux, ici la prise entrée.

Le processeur 75 collecte l'ensemble des messages d'état de chacune des pièces 101 à 109 de l'assemblage.

Le processeur 75 calcule, à partir des informations tirées des messages d'état des pièces de revêtement 101 à 109, une cartographie ou matrice d'état 91 des capteurs de pression 23 des pièces de revêtement 101 à 109 interconnectées. Une cartographie 91 des états de chacun des capteurs de pression 23 de chacune des pièces de revêtement 101 à 109 de la figure 9 est représentée schématiquement en figure 11.

Les espaces marqués d'une croix représentent un capteur de pression 23 actif, c'est-à-dire détectant une pression supérieure à un seuil de détection choisi. Les espaces vides représentent des capteurs de pression 23 inactifs, c'est-à-dire détectant une pression inférieure au seuil de détection prédéfini.

Les calculs nécessaires à la détection peuvent être réalisés au sein de l'unité centrale 71. Pour cela, cette dernière peut comprendre une unité de calcul sous la forme d'un dispositif embarqué, d'une carte dédiée ou de tout autre moyen approprié. L'unité centrale 71 peut également comprendre des moyens de communication, filaires (par ligne téléphonique classique ou par réseau, par exemple Ethernet), ou sans fil (par le biais d'une interface de communication ou module GSM, GPRS, 3G, ou Wifi). Le module de communication est relié à la sortie du processeur 75.

En outre, l'unité centrale 71 peut être réalisée en plusieurs parties. Dans ce cas, l'unité centrale 71 comprend une première partie incluant le processeur 75, reliée aux capteurs de pressions 23, et qui comprend une interface de communication similaire à celle décrite plus haut.

La première partie communique avec une seconde partie déportée, qui peut réaliser les calculs de détection mentionnés dans la suite, et comprendre elle-même une interface de communication similaire à celle décrite plus haut.

Ces interfaces ou modules de communications peuvent être utilisées pour émettre des alertes en cas de détection de chute, par exemple vers un serveur central, un central de télésurveillance, vers un centre d'appel de secours, vers le poste d'infirmerie dans le cas d'un hôpital, d'une clinique ou d'une maison de retraite, etc.

Enfin, l'unité centrale 71 peut inclure uniquement une interface de communication similaire à celle décrite plus haut, l'ensemble des calculs de détection de chute étant déporté sur un serveur de détection auquel est reliée l'unité centrale 71 via cette interface.

La figure 12 représente un exemple de diagramme de flux que l'unité de calcul de détection de chute peut mettre en oeuvre.

Dans une opération 300, l'unité de calcul est initialisée, avec l'ensemble des paramètres liés à la détection de chutes, et avec l'initialisation de l'interface de communication.

Ensuite dans une opération 310, une boucle de détection commence. Cette boucle comprend la collecte des états des capteurs de pression 23 des pièces 101 à 109, c'est-à-dire les données correspondant à la matrice d'état 91.

Ensuite, dans une opération 320, l'unité de calcul vérifie la liste des identifiants de capteurs de pression 23, des identifiants de localisation des pièces et des marqueurs de temps, afin de déterminer si ceux-ci vérifient l'une des conditions de détection de chute décrites par la suite.

Si c'est le cas, alors l'interface de communication est activée dans une opération 330 pour envoyer un signal de détection de chute, puis la détection reprend avec l'opération 310. Sinon, la boucle reprend directement avec l'opération 310.

L'envoi du signal de détection de chute peut comprendre toute information utile, y compris l'emplacement de la chute déduite de l'identification des capteurs de pression 23 en jeu et de la ou les pièce(s) 101 à 109 les comprenant, une durée associée aux marqueurs de temps afin d'indiquer l'heure de la chute, etc.

Lorsqu'une personne chute, elle se retrouve nécessairement allongée sur le dos, sur le ventre, ou du moins avec une partie assez étendue de son corps par terre. Un maillage suffisamment serré permet alors de détecter la différence entre une chute et la présence d'une ou plusieurs personnes debout sur l'ensemble de pièces de revêtement 101 à 109.

Le maillage de l'exemple décrit en référence aux figures 7 et 8 est serré, ce qui offre une grande précision. En effet, l'étendue d'une personne couchée signifie que l'on peut détecter une chute :
- lorsque plus de dix points de détection, c'est-à-dire de capteurs de pression 23, sont activés dans un carré d'environ 30 cm de côté, ou dans un rectangle de surface similaire et dont la diagonale est d'environ 35 cm, soit sur une surface d'environ 0,09m², pendant une durée minimale, par exemple de l'ordre de une minute, ou
- lorsque quatre points de détection alignés dans une première direction principale, en diagonale ou dans une seconde direction principale sont activés pendant une durée minimale, par exemple de l'ordre d'une minute.

D'une manière générale, la durée minimale pour la détection peut être choisie supérieure à 15 secondes. Dans une variante, la détection peut ne pas dépendre d'une durée minimale.

En fonction de l'agencement des capteurs dans les pièces et notamment leur espacement mutuel, l'algorithme de calcul est adapté, par exemple pour pouvoir choisir la précision de la mesure.

Ces scénarios excluent en effet le cas de la marche ou de la présence de plusieurs personnes sur le sol revêtu des pièces 101 à 109. En effet, un pied adulte est dans la très grande majorité des cas d'une longueur inférieure à 35 cm, qui correspond à une pointure 53. Par conséquent, les critères de détection décrits ci-dessus permettent de discriminer la position debout, dans laquelle seuls les pieds sont au contact du sol. De plus, dans le cas où plusieurs personnes sont présentes, même si elles sont très proches, elles ne provoqueront pas de détection grâce aux maillages décrits, même si la distance centre à centre des évidements 47 est de 20 cm.

Dans une variante, l'unité de calcul peut être paramétrée pour déclencher un signal d'alerte spécifique en cas de détection d'un individu debout ou en marche. L'invention permet alors, outre la détection de chutes, de détecter une intrusion ou de manière générale la présence et la localisation d'une personne, qu'elle soit debout ou non. L'analyse de telles données a posteriori est rendue précise et efficace lorsqu'elle est couplée à un système de stockage de données. Dans le contexte de maisons de santé accueillant des personnes âgées, un tel paramétrage permet par exemple de détecter et localiser rapidement une personne égarée dans l'établissement. Ce type de fonction est particulièrement utile pour les personnes atteintes de troubles de la mémoire.

Dans une autre variante, l'activation et/ou la désactivation de certaines fonctions, par exemple celles décrites au paragraphe précédent, peuvent être automatisées. Par exemple, le déclenchement d'une alerte en cas de personne debout peut n'être activé que durant des plages horaires prédéfinies telles que la nuit.

De telles pièces de revêtement permettent, tout en étant conçues et fabriquées avec des processus industriels, d'offrir une bonne adaptabilité au moment de l'installation dans les espaces à équiper. Des chambres ou couloirs de maisons de retraites peuvent être équipés en utilisant les pièces de revêtements précédemment décrites et en adaptant leur nombre et leur agencement mutuel, comme il est connu de le faire pour du carrelage par exemple. Ici, l'interconnexion des pièces de revêtement permet de créer un réseau de capteurs selon de très nombreuses combinaisons. Le nombre de pièces de revêtement nécessaire est sensiblement proportionnel à la surface totale à couvrir. D'autre part, l'assemblage des pièces de revêtement entre elles est facilité ce qui réduit les risque d'erreurs et de malfaçons à la pose.

Dans les exemples décrits précédemment, les solutions proposées étaient choisies en privilégiant l'homogénéité des pièces de revêtement utilisées de sorte à faciliter le travail des opérateurs effectuant l'installation de ces revêtements et à limiter les risques d'erreurs lors de l'installation. Par exemple, il est facile d'installer et de combiner un ensemble de pièces sensiblement rectangulaires dont chacun des côtés est muni d'une prise.

En fonction des circonstances, il peut être préférable de minimiser les coûts de fabrication des pièces et/ou d'affiner l'adéquation des pièces de revêtements avec les surfaces à couvrir. À cet effet, des pièces spécifiques peuvent être adaptées pour une utilisation spécifique. Par exemple, des pièces de revêtement rectangulaires peuvent être agencées privées de prise sur l'un ou deux des côtés pour une installation le long d'un mur ou dans un coin. Pour les mêmes raisons, les pièces de revêtement peuvent toutes être munies de seulement deux prises de sortes à définir une unique entrée et une unique sortie et donc un unique cheminement de proche en proche des informations. Dans ce cas, la disposition mutuelle des prises d'une pièce et la disposition mutuelle des pièces d'un assemblage sont dépendantes et nécessitent une adaptation au cas par cas.

L'invention ne se limite pas aux exemples de pièces de revêtement décrites ci-avant, seulement à titre d'exemples, mais elle englobe toutes les variantes que pourra envisager l'homme de l'art dans le cadre des revendications ci-après.

L'invention porte également sur des kits de montage comprenant une pluralité de pièces de revêtement ou dalles telles que décrites précédemment. Un tel kit d'installation peut en outre comprendre une unité centrale telle que décrite précédemment.

L'invention concerne aussi le procédé de détection de chute comprenant :
- relever l'état d'un ensemble de capteurs de pression disposés dans une pluralité de pièces de revêtement reliées entre elles,
- transmettre des données d'état des capteurs tirées de la relève de la pluralité de pièces de revêtement à une unité centrale,
- détecter pendant une durée nominale choisie, l'une au moins de ces conditions :
   - des capteurs de pressions à l'état "soumis à une pression" contigus correspondent à une surface supérieure à une valeur seuil prédéfinie, ou
   - des données de localisations associées à des capteurs de pressions à l'état "soumis à une pression" indiquent un alignement continu de capteurs dont les deux capteurs d'extrémité sont éloignés d'une distance supérieure à un seuil prédéfini,
   - émettre un signal d'alerte en réponse à la détection de l'une de ces deux conditions.

## Revendications

1. Pièce (1) de revêtement pour la détection de chute comprenant
- un corps (3) délimité par des bords (5, 7, 9, 11),
- une pluralité de capteurs de pression (23) répartis selon une géométrie choisie dans le corps (3),
- une unité de traitement (27) reliée à certains au moins des capteurs de pression (23) et agencée pour collecter des informations d'état de chacun de ces capteurs de pression (23), et
- une première prise (15, 17, 21) et une seconde prise (19), chacune reliée à l'unité de traitement (27), disposée au voisinage d'un bord (5, 7, 9, 11) et agencée pour pouvoir être reliée à une prise d'une autre pièce similaire,
l'unité de traitement (27) étant agencée pour
associer des informations de localisation tirées desdites informations d'état à des informations de localisation de la pièce (1),
recevoir, par l'intermédiaire de la première prise (15, 17, 21), des informations en provenance d'une première autre pièce similaire, et
émettre, par l'intermédiaire de la seconde prise (19), les informations associées et/ou lesdites informations reçues vers une seconde autre pièce similaire.

2. Pièce selon la revendication 1, dans laquelle l'unité de traitement (27) est agencée pour émettre les informations associées et les informations reçues à des intervalles de temps prédéfinis.

3. Pièce selon l'une des revendications précédentes, dans laquelle l'unité de traitement (27) est agencée pour émettre les informations associées en réponse à des modifications d'état des capteurs de pression (23).

4. Pièce selon l'une des revendications précédentes, dans laquelle le corps (3) comprend une couche de surface (41) et une couche inférieure (45), une couche intermédiaire (43) étant disposée entre la couche de surface (41) et la couche inférieure (45) et comprenant une pluralité d'évidements (47) logeant au moins en partie les capteurs de pression (23).

5. Pièce selon la revendication 4, dans laquelle la couche de surface (41) et la couche inférieure (45) comprennent chacune un film portant, sur des faces en regard, un jeu de lames conductrices (31 ; 33), les lames conductrices (31 ; 33) étant sensiblement parallèles au sein de chaque jeu, et les jeux de lames conductrices (31 ; 33) étant agencés de sorte que les lames conductrices (31 ; 33) de jeux distincts soient sensiblement perpendiculaires entre elles et se croisent au niveau des évidements (47) de la couche intermédiaire (43), de manière à former à chaque fois un capteur de pression (23).

6. Pièce selon l'une des revendications précédentes, dans laquelle le corps (3) est de forme générale rectangulaire ou carrée, l'unité de traitement (27) étant disposée dans un coin du corps (3).

7. Pièce selon l'une des revendications précédentes, dans laquelle l'unité de traitement (27) est en outre agencée pour calculer des informations de localisation de pièce en fonction d'autres informations de localisation.

8. Pièce selon la revendication 7, dans laquelle l'unité de traitement (27) est agencée pour calculer les informations d'identification de pièces similaires en fonction d'informations d'identification de la pièce (1) de manière itérative.

9. Pièce selon l'une des revendications précédentes, dans laquelle le corps (3) présente une forme aplatie et dont la surface est comprise entre 400 cm² et 2,25 m².

10. Pièce selon l'une des revendications précédentes, dans laquelle la première prise (15, 17, 21) et la seconde prise (19) sont mutuellement agencées de sorte à former un détrompeur pour l'installation de la pièce.

11. Kit d'installation de revêtement pour la détection de chute comprenant une pluralité de pièces (101 ; 102 ; 103 ; 104) selon l'une des revendications précédentes.

12. Kit selon la revendication 11, comprenant en outre une unité centrale (71) pour la détection de chute incluant
- une prise (73) propre à être reliée à au moins une prise (15, 17, 19, 21) d'au moins une pièce (101),
- un processeur (75) relié à ladite prise (73) et agencé pour
- collecter des informations d'état de capteurs de pression (23) associées à des informations de localisation desdits capteurs de pression (23) couplées à des informations d'identification de pièces (101 ; 102 ; 103 ; 104),
- calculer à partir des informations collectées une matrice d'état (91) d'un ensemble de pièces (101 ; 102 ; 103 ; 104) interconnectées,
- détecter un état d'alerte à partir de la matrice d'état (91),
- émettre un signal d'alerte sélectionné en fonction de l'état d'alerte détecté.

13. Kit selon la revendication 12, comprenant en outre un module de communication relié à la sortie du processeur (75) et agencé pour transmettre le signal d'alerte à un serveur central.

14. Kit selon l'une des revendications 11 à 13, dans lequel le processeur (75) est agencé pour ignorer des informations d'état de certains au moins des capteurs de pression (23).

## Patentansprüche

1. Belagelement (1) zur Sturzerkennung, Folgendes aufweisend:
- einen Körper (3), der durch Ränder (5, 7, 9, 11) begrenzt ist,
- mehrere Drucksensoren (23), die nach einer ausgewählten Geometrie im Körper (3) verteilt sind,
- eine Verarbeitungseinheit (27), die an zumindest manche der Drucksensoren (23) angeschlossen und dazu eingerichtet ist, Zustandsinformationen über jeden dieser Drucksensoren (23) zu sammeln, und
- eine erste Anschlussstelle (15, 17, 21) und eine zweite Anschlussstelle (19), wovon jede an die Verarbeitungseinheit (27) angeschlossen, im Nahbereich eines Rands (5, 7, 9, 11) angeordnet und dazu eingerichtet ist, an eine Anschlussstelle eines anderen, ähnlichen Elements angeschlossen werden zu können,
wobei die Verarbeitungseinheit (27) dazu eingerichtet ist, aus den Zustandsinformationen abgeleitete Lokalisierungsinformationen mit Lokalisierungsinformationen des Elements (1) zu assoziierten,
mittels der ersten Anschlussstelle (15, 17, 21), von einem ersten anderen, ähnlichen Element stammende Informationen zu empfangen,
und
mittels der zweiten Anschlussstelle (19) die assoziierten Informationen und/oder die empfangenen Informationen an ein zweites anderes, ähnliches Element abzugeben.

2. Element nach Anspruch 1, wobei die Verarbeitungseinheit (27) dazu eingerichtet ist, die assoziierten Informationen und die empfangenen Informationen in vordefinierten Zeitintervallen abzugeben.

3. Element nach einem der vorhergehenden Ansprüche, wobei die Verarbeitungseinheit (27) dazu eingerichtet ist, die assoziierten Informationen in Antwort auf Zustandsänderungen der Drucksensoren (23) abzugeben.

4. Element nach einem der vorhergehenden Ansprüche, wobei der Körper (3) eine Oberflächenschicht (41) und eine untere Schicht (45) umfasst, wobei eine Zwischenschicht (43) zwischen der Oberflächenschicht (41) und der unteren Schicht (45) angeordnet ist und mehrere Ausnehmungen (47) hat, die zumindest zum Teil die Drucksensoren (23) aufnehmen.

5. Element nach Anspruch 4, wobei die Oberflächenschicht (41) und die untere Schicht (45) jeweils eine Folie haben, die an gegenüberliegenden Flächen einen Satz leitfähiger Leisten (31; 33) tragen, wobei die leitfähigen Bänder (31; 33) im Wesentlichen parallel innerhalb eines jeden Satzes sind, und wobei die Sätze leitfähiger Bänder (31; 33) so eingerichtet sind, dass die leitfähigen Bänder (31; 33) unterschiedlicher Sätze zueinander im Wesentlichen senkrecht sind und sich im Bereich der Ausnehmungen (47) der Zwischenschicht (43) kreuzen, um jedes Mal einen Drucksensor (23) zu bilden.

6. Element nach einem der vorhergehenden Ansprüche, wobei der Körper (3) von allgemein rechteckiger oder quadratischer Form ist, wobei die Verarbeitungseinheit (27) in einer Ecke des Körpers (3) angeordnet ist.

7. Element nach einem der vorhergehenden Ansprüche, wobei die Verarbeitungseinheit (27) darüber hinaus dazu eingerichtet ist, Elementlokalisierungsinformationen in Abhängigkeit von anderen Lokalisierungsinformationen zu berechnen.

8. Element nach Anspruch 7, wobei die Verarbeitungseinheit (27) dazu eingerichtet ist, Identifizierungsinformationen von ähnlichen Elementen in Abhängigkeit von Identifizierungsinformationen des Elements (1) auf iterative Weise zu berechnen.

9. Element nach einem der vorhergehenden Ansprüche, wobei der Körper (3) eine abgeflachte Form aufweist, deren Oberfläche zwischen 400 cm² und 2,25 m² beträgt.

10. Element nach einem der vorhergehenden Ansprüche, wobei die erste Anschlussstelle (15, 17, 21) und die zweite Anschlussstelle (19) gegenseitig so eingerichtet sind, dass sie eine Unverwechselbarkeitseinrichtung zum Einbau des Elements bilden.

11. Bausatz zum Verlegen eines Belags zur Sturzerkennung, mehrere Elemente (101; 102; 103; 104) nach einem der vorhergehenden Ansprüche umfassend.

12. Bausatz nach Anspruch 11, darüber hinaus eine Zentraleinheit (71) zur Sturzerkennung umfassend, Folgendes enthaltend:
- eine Anschlussstelle (73), die sich dazu eignet, an mindestens eine Anschlussstelle (15, 17, 19, 21) mindestens eines Elements (101) angeschlossen zu werden,
- einen Prozessor (75), der an die Anschlussstelle (73) angeschlossen und dazu eingerichtet ist
- mit Lokalisierungsinformationen der Drucksensoren (23) assoziierte Zustandsinformationen von Drucksensoren (23) verbunden mit Identifizierungsinformationen von Elementen (101; 102; 103; 104) zu sammeln,
- aus den gesammelten Informationen eine Zustandsmatrix (91) einer Einheit von miteinander verbundenen Elementen (101; 102; 103; 104) zu berechnen,
- einen Alarmzustand aus der Zustandsmatrix (91) zu erfassen,
- ein in Abhängigkeit vom erfassten Alarmzustand ausgewähltes Alarmsignal abzugeben.

13. Bausatz nach Anspruch 12, darüber hinaus ein Kommunikationsmodul umfassend, das an den Ausgang des Prozessors (75) angeschlossen und dazu eingerichtet ist, das Alarmsignal an einen zentralen Server zu übertragen.

14. Bausatz nach einem der Ansprüche 11 bis 13, wobei der Prozessor (75) dazu eingerichtet ist, Zustandsinformationen von zumindest manchen der Drucksensoren (23) zu ignorieren.

## Claims

1. Covering component (1) for detecting falls comprising:
- a body (3) which is delimited by edges (5, 7, 9, 11),
- a plurality of pressure sensors (23) which are distributed in accordance with a selected geometry in the body (3),
- a processing unit (27) which is connected to at least some of the pressure sensors (23) and which is arranged to collect status information of each of these pressure sensors (23), and
- a first socket (15, 17, 21) and a second socket (19), each of which is connected to the processing unit (27) and arranged in the region of an edge (5, 7, 9, 11), and arranged so as to be connectable to a socket of another similar component,
the processing unit (27) being arranged so as to associate location information which is derived from the status information with location information of the component (1),
receive, via the first socket (15, 17, 21), information originating from a first other similar component, and
transmit, via the second socket (19), the associated information and/or the received information to a second other similar component.

2. Component according to claim 1, wherein the processing unit (27) is arranged to transmit the associated information and the received information at predefined time intervals.

3. Component according to either of the preceding claims, wherein the processing unit (27) is arranged so as to transmit associated information in response to status modifications of the pressure sensors (23).

4. Component according to any one of the preceding claims, wherein the body (3) comprises a surface layer (41) and a lower layer (45), an intermediate layer (43) being arranged between the surface layer (41) and the lower layer (45) and comprising a plurality of recesses (47) which at least partially accommodate the pressure sensors (23).

5. Component according to claim 4, wherein the surface layer (41) and the lower layer (45) each comprise a film which carries, on opposing faces, a set of conductive strips (31; 33), the conductive strips (31; 33) being substantially parallel within each set, and the sets of conductive strips (31; 33) being arranged so that the conductive strips (31; 33) of separate sets are substantially perpendicular to each other and cross each other in the region of the recesses (47) of the intermediate layer (43), in order to form a pressure sensor (23) each time.

6. Component according to any one of the preceding claims, wherein the body (3) is generally rectangular or square, the processing unit (27) being arranged in a corner of the body (3).

7. Component according to any one of the preceding claims, wherein the processing unit (27) is further arranged to calculate component location information in accordance with other location information.

8. Component according to claim 7, wherein the processing unit (27) is arranged in order to calculate the identification information of similar components in accordance with identification information of the component (1) in an iterative manner.

9. Component according to any one of the preceding claims, wherein the body (3) has a flattened shape whose surface-area is between 400 cm² and 2.25 m².

10. Component according to any one of the preceding claims, wherein the first socket (15, 17, 21) and the second socket (19) are mutually arranged so as to form a failsafe for the installation of the component.

11. Covering installation kit for the detection of falls comprising a plurality of components (101; 102; 103; 104) according to any one of the preceding claims.

12. Kit according to claim 11, further comprising a central unit (71) for the detection of a fall, including:
- a socket (73) which is capable of being connected to at least one socket (15, 17, 19, 21) of at least one component (101),
- a processor (75) which is connected to the socket (73) and which is arranged so as to
- collect from pressure sensors (23) status information which is associated with location information of the pressure sensors (23) which is linked to component identification information (101; 102; 103; 104),
- calculate from the information collected a status matrix (91) of an assembly of interconnected components (101; 102; 103; 104),
- detect an alert status from the status matrix (91),
- transmit an alert signal which is selected in accordance with the alert status detected.

13. Kit according to claim 12, further comprising a communication module which is connected to the output of the processor (75) and which is arranged so as to transmit the alert signal to a central server.

14. Kit according to any one of claims 11 to 13, wherein the processor (75) is arranged so as to ignore the status information of at least some of the pressure sensors (23).
